# FASCICULE DE BREVET EUROPEEN

(11) **EP 2 719 689 B1**
(45) Date de publication et mention de la délivrance du brevet: **23.12.2015**
(21) Numéro de dépôt: 13188228.4
(22) Date de dépôt: 11.10.2013
(51) Int. Cl.: C07D 223/14, C07C 253/30, C07C 255/37

(54) **Nouveau procede de synthese du 3-(2-bromo-4,5-dimethoxphenyl)propanenitrile, et application a la synthese de l'ivabradine et de ses sels d'addition a un acide pharmaceutiquement acceptable**
Neues Syntheseverfahren von 3-(2-Brom-4,5-Dimethoxyphenyl)Propanenitril, und Anwendung zur Synthese von Ivabradin und seinen Additionssalzen zu einer pharmazeutisch akzeptierbaren Säure
Novel method for synthesising 3-(2-bromo-4,5-dimethoxyphenyl) propanenitrile and use for synthesising ivabradine and the added salts thereof with a pharmaceutically acceptable acid

(30) Priorité: 12.10.2012 FR 1259745
(43) Date de publication de la demande: 16.04.2014
(73) Titulaire: Les Laboratoires Servier, 92284 Suresnes Cedex (FR)
(72) Inventeur: Carranza, Maria del Pilar, 13670 Villarrubia de los Ojos (ES); Garcia Aranda, Maria Isabel, 45006 Toledo (ES); Gonzalez, José Lorenzo, 45007 Toledo (ES); Sanchez, Frédéric, 45111 Cobisa (ES)

(56) Documents cités:
- WO-A1-2011/008597
- WO-A1-2011/138625
- DE-A1- 2 303 919
- US-A- 4 618 683
- ZHAO SHENG YIN ET AL: "A practical synthesis of 1-cyano-4,5-dimethoxybenzocyclobutene", JOURNAL OF CHEMICAL RESEARCH, SCIENCE REVIEWS LTD, GB, no. 7, 1 janvier 2009 (2009-01-01), pages 420-422, XP008162622, ISSN: 0308-2342
- PAULL K D ET AL: "A facile synthesis of 4-substituted 3a,4,5,9b-tetrahydrobenz(e)isoindolines", JOURNAL OF ORGANIC CHEMISTRY, ACS, US, vol. 37, no. 21, 1 janvier 1972 (1972-01-01), pages 3374-3376, XP002312221, ISSN: 0022-3263, DOI: 10.1021/JO00986A050
- SHANINA D. SANDERS ET AL: "Total synthesis of (+)-virgatusin via AlCl3-catalyzed [3+2] cycloaddition", CHEMICAL COMMUNICATIONS, no. 34, 1 janvier 2009 (2009-01-01), page 5135, XP055068847, ISSN: 1359-7345, DOI: 10.1039/b911765b -& Sd Sanders: "Supplementary Material / Total Synthesis of (+)-Virgatusin via AlCl3 -Catalyzed [3 + 2] Cycloaddition", Chemical Communications, Supplementary Material, 1 janvier 2009 (2009-01-01), pages S1-S30, XP055068856, Extrait de l'Internet: URL:http://www.rsc.org/suppdata/cc/b9/b911 765b/b911765b.pdf [extrait le 2013-07-01]

## Description

La présente invention concerne un procédé de synthèse du (3-(2-bromo-4,5-diméthoxyphényl)propanenitrile de formule (I) : 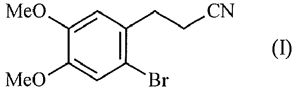 et son application à la synthèse de l'ivabradine et de ses sels d'addition à un acide pharmaceutiquement acceptable.

Le composé de formule (I) obtenu selon le procédé de l'invention est utile dans la synthèse de l'ivabradine de formule (II) : 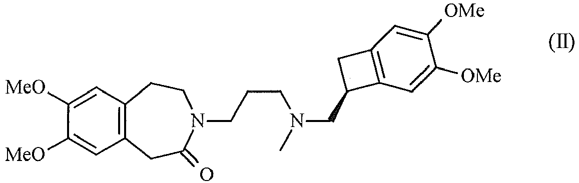 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut être transformée en un de ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique et en un de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

Le sel d'addition à un acide pharmaceutiquement acceptable de l'ivabradine peut être préparé à partir d'un acide choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la préparation de l'ivabradine à partir du 3,4-diméthoxybicyclo[4.2.0]octa-1,3,5-triène-7-carbonitrile de formule (III) : 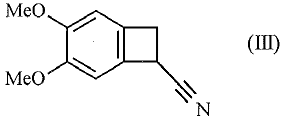 qui est transformé en composé de formule (IV) : 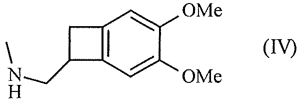 qui est dédoublé pour conduire au composé de formule (V) : 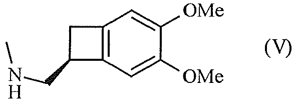 qui est mis en réaction avec le composé de formule (VI) : 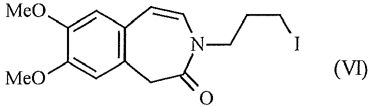 pour conduire au composé de formule (VII) : 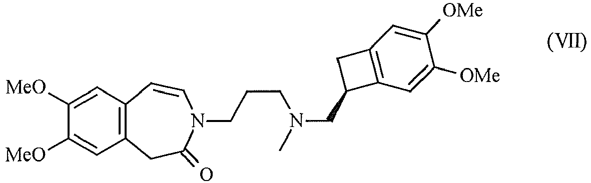 dont l'hydrogénation catalytique conduit à l'ivabradine, qui est alors transformée en son chlorhydrate.

La préparation du composé de formule (III) à partir du composé de formule (I) est décrite dans Tetrahedron 1973, 29, pp 73-76.

Ce même document décrit également une voie de synthèse du composé de formule (I) à partir du 2-bromo-4,5-diméthoxybenzaldéhyde en trois étapes avec un rendement global de 65%.

La préparation du composé de formule (I) par une réaction de bromation du 3-(3,4-diméthoxyphényl)propanenitrile en présence de dibrome dans l'acide acétique est décrite dans J. Org. Chem 1972, vol. 37, n°21, pp 3374-3376 avec un rendement de 48%.

Plus récemment, Zhao et al. ont décrit une synthèse du composé de formule (I) à partir du 3,4-diméthoxybenzaldéhyde en trois étapes avec 51% de rendement global (CN101 407 474 A et J. Chem. Res. 2009, 7, pp 420-422).

Le composé de formule (I) est un intermédiaire clé dans la synthèse de l'ivabradine.

Compte-tenu de l'intérêt industriel de l'ivabradine et de ses sels, il était impératif de trouver un procédé performant permettant d'accéder au (3-(2-bromo-4,5-diméthoxyphényl)propanenitrile de formule (I) avec un excellent rendement.

La présente invention concerne un procédé de synthèse du composé de formule (I) : 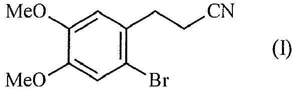 caractérisé en ce que le composé de formule (VIII) : 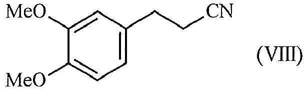 est soumis à l'action du *N*-bromosuccinimide en présence d'un solvant organique pour conduire au composé de formule (I).

Parmi les solvants organiques pouvant être utilisés pour effectuer la transformation du composé de formule (VIII) en composé de formule (I), on peut citer à titre non limitatif le *N*,*N*-diméthylformamide, le tétrahydrofurane, l'acétonitrile, l'acide acétique, le méthanol, le dichlorométhane et le toluène.

Le solvant préférentiellement utilisé pour effectuer la transformation du composé de formule (VIII) en composé de formule (I) est le *N*,*N*-diméthylformamide.

La transformation du composé de formule (VIII) en composé de formule (I) est conduite à une température préférentiellement comprise entre -10°C et 30°C.

La présente invention concerne également un procédé de synthèse du composé de formule (I) à partir du composé de formule (VIII), caractérisé en ce que ledit composé de formule (VIII) est préparé à partir du composé de formule (IX) : 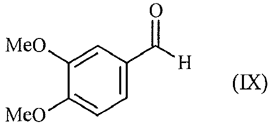 qui est transformé en composé de formule (X) : 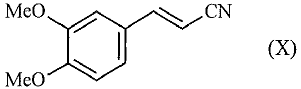 en présence d'un ylure de phosphore et d'une base dans un solvant organique,
lequel est transformé en composé de formule (VIII) : 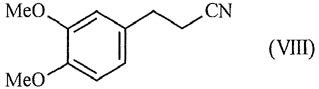 par une réaction de réduction en présence d'un agent donneur d'hydrure dans un solvant organique ou un mélange de solvants organiques,
lequel est transformé en produit de formule (I) : 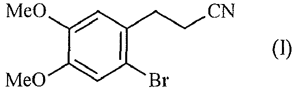 selon le procédé décrit plus haut.

Parmi les ylures de phosphore pouvant être utilisés pour effectuer la transformation du composé de formule (IX) en composé de formule (X), on peut citer à titre non limitatif le diéthylcyanométhylphosphonate et le (triphénylphosphoranylidène)acétonitrile.

L'ylure de phosphore préférentiellement utilisé pour effectuer la transformation du composé de formule (IX) en composé de formule (X) est le diéthylcyanométhylphosphonate.

Parmi les bases pouvant être utilisées pour effectuer la transformation du composé de formule (IX) en composé de formule (X), on peut citer à titre non limitatif le *tert*-butylate de potassium, l'hydrure de sodium, la triéthylamine et l'hydrogénocarbonate de potassium.

La base préférentiellement utilisée pour effectuer la transformation du composé de formule (IX) en composé de formule (X) est le *tert*-butylate de potassium.

Parmi les solvants organiques pouvant être utilisés pour effectuer la transformation du composé de formule (IX) en composé de formule (X), on peut citer à titre non limitatif le tétrahydrofurane, l'acétonitrile et le toluène.

Le solvant organique préférentiellement utilisé pour effectuer la transformation du composé de formule (IX) en composé de formule (X) est le tétrahydrofurane.

La transformation du composé de formule (IX) en composé de formule (X) est préférentiellement conduite à une température comprise entre -5°C et 120°C.

Parmi les agents donneurs d'hydrure pouvant être utilisés pour effectuer la transformation du composé de formule (X) en composé de formule (VIII), on peut citer à titre non limitatif le tétraborohydrure de sodium, le formiate d'ammonium en présence de Pd/C et l'acide formique en présence de Pd(OAc)₂.

L'agent donneur d'hydrure préférentiellement utilisé pour effectuer la transformation du composé de formule (X) en composé de formule (VIII) est le tétraborohydrure de sodium. Parmi les solvants organiques pouvant être utilisés pour effectuer la transformation du composé de formule (X) en composé de formule (VIII), on peut citer à titre non limitatif les solvants alcooliques, tels que le méthanol et l'éthanol, le tétrahydrofurane.

Un mélange de solvants organiques peut également être utilisé pour effectuer la transformation du composé de formule (X) en composé de formule (VIII), préférentiellement le mélange pyridine/méthanol.

La transformation du composé de formule (X) en composé de formule (VIII) est préférentiellement conduite à une température comprise entre 25°C et 110°C.

La présente invention concerne également un procédé de synthèse de l'ivabradine à partir du composé de formule (I) préparé selon le procédé de l'invention et transformé en composé de formule (III) en suivant l'enseignement de l'art antérieur (Tetrahedron 1973, 29, pp 73-76) par une réaction de cyclisation intramoléculaire en milieu basique ; ledit composé de formule (III) étant ensuite transformé en ivabradine selon le procédé décrit dans EP 0 534 859.

Les exemples suivants illustrent l'invention.

Les points de fusion ont été mesurés avec BÜCHI Melting Point B-545 (Volt. 230VAC, Freq. 50/60 Hz, Power max. 220W).

### Liste des abréviations utilisées

DMF : *N*,*N*-diméthylformamide
HPLC : High Performance Liquid Chromatography
NBS : *N*-bromosuccinimide
P.F. : point de fusion
THF : tétrahydrofurane

### Préparation A : (2E)-3-(3,4-diméthoxyphényl)prop-2-ènenitrile

7 g de 3,4-diméthoxybenzaldéhyde (42,1 mmoles) sont dissous dans 84 mL de THF et la solution est refroidie à 0°C. 8,2 g de diéthylcyanométhylphosphonate (7,5 mL, 46,3 mmoles, 1,1 éq.) puis ensuite 5,2 g de *tert*-butoxyde de potassium (46,3 mmoles, 1,1 éq.) sont ajoutés petit à petit. Le mélange est agité une heure à 0°C puis pendant une nuit à température ambiante. Le milieu réactionnel est hydrolysé par 175 mL d'eau et extrait deux fois au dichlorométhane. Les phases organiques sont rassemblées et le solvant est évaporé sous pression réduite pour conduire à 7,69 g de produit du titre sous forme d'un solide beige.
Rendement = 96,5%
P.F. = 93-98°C

### Préparation B : 3-(3,4-diméthoxyphényl)propanenitrile

A une solution de 1 g (5,3 mmoles) de (2*E*)-3-(3,4-diméthoxyphényl)prop-2-ènenitrile dans 9,3 mL de pyridine et 2,8 mL de méthanol est ajouté petit à petit 0,24 g de NaBH₄ (6,3 mmol, 1,2 éq.). Le milieu réactionnel est chauffé à reflux (100°C) pendant 9 heures. Après refroidissement à température ambiante, le milieu réactionnel est additionné à une solution de 9 mL d'acide chlorhydrique à 37% dans 24 g de glace. La solution est extraite deux fois au dichlorométhane. Les phases organiques sont rassemblées et le solvant est évaporé sous pression réduite pour conduire à 0,82 g d'une huile brun rouge qui cristallise.
Rendement = 82%
P.F. = 45-48°C

### Exemple 1 : 3-(2-bromo-4,5-diméthoxyphényl)propanenitrile

Une solution de 1 g (5,3 mmoles) de 3-(3,4-diméthoxyphényl)propanenitrile dans 42 mL de DMF est refroidie à 0°C. A cette solution est ajouté petit à petit 0,93 g de NBS (5,2 mmoles, 1 éq.). Après 30 minutes d'agitation à 0°C, le milieu réactionnel est ramené à température ambiante et agité pendant deux heures. Il est ensuite hydrolysé par 170 mL d'eau et extrait avec 2 fois 130 mL d'acétate d'éthyle. Les phases organiques sont rassemblées puis lavées d'abord avec une solution aqueuse saturée en Na₂S₂O₅ puis avec de l'eau. Après évaporation du solvant sous pression réduite, 1,29 g d'une huile incolore cristallisant sous forme de solide blanc sont obtenus.
Rendement = 98%
Pureté (HPLC) : 96,8%
P.F. = 78-80°C

### Exemple 2 : 3,4-diméthoxybicyclo[4.2.0]octa-1,3,5-triène-7-carbonitrile D'après Tetrahedron 1973, 29, pp 73-76

A une solution de NaNH₂, préparée à partir de 200 mL de NH₃ liquide et d'1 g de Na (catalyseur FeCl₃), sont ajoutés par portions 5,4 g de 3-(2-bromo-4,5-diméthoxyphényl)propanenitrile et le mélange réactionnel est agité à température ambiante pendant 2 heures. Après évaporation de l'excès de NH₃, 2 g de NH₄Cl et 200 mL d'eau sont ajoutés par portions. Les cristaux gris formés sont collectés et recristallisés dans l'éthanol pour conduire à 2,38 g du produit attendu
Rendement = 74%
P.F. = 84-85°C

### Exemple 3 : 3,4-diméthoxy-N-méthylbicyclo[4.2.0]octa-1,3,5-trién-7-amine D'après EP 0 534 859

Stade 1 : chlorhydrate de 3,4-diméthoxybicyclo[4.2.0]octa-1,3,5-trién-7-amine On ajoute goutte à goutte et sous agitation à température ambiante, 312 mL d'une solution molaire de borane complexé avec du THF à une solution de 25 g de 3,4-diméthoxybicyclo[4.2.0]octa-1,3,5-triène-7-carbonitrile dans 250 mL de THF. On laisse en contact pendant 12 heures, puis on ajoute 200 mL d'éthanol et on agite 1 heure. On ajoute, goutte à goutte, 100 mL d'éther chlorhydrique 3,3N. On obtient 27,7 g du produit attendu.
Rendement = 90%
P.F. = 205°C

Stade 2 : (3,4-diméthoxybicyclo[4.2.0]octa-1,3,5-trién-7-yl)carbamate d'éthyle
On coule 1,5 mL de chloroformiate d'éthyle sur une suspension de 3,4 g du composé obtenu au stade 1 de 4,5 mL de triéthylamine et de 50 mL de dichlorométhane. On laisse une nuit sous agitation à température ambiante puis on lave à l'eau et à l'acide chlorhydrique 1N. On sèche et on évapore à sec le solvant. On obtient 3,2 g d'une huile correspondant au produit attendu.
Rendement = 80%

Stade 3 : 3,4-diméthoxy-*N*-méthylbicyclo[4.2.0]octa-1,3,5-trién-7-amine
On ajoute 3,2 g du composé obtenu au stade 2 en solution dans 30 mL de THF à une suspension de 0,9 g de LiAlH₄ dans 20 mL de THF. On porte au reflux 1 heure 30 minutes puis on hydrolyse par 0,6 ml d'eau et 0,5 mL de soude à 20%, et enfin par 2,3 mL d'eau. Les sels minéraux sont ensuite filtrés, rincés au THF puis le filtrat obtenu est évaporé à sec. On obtient 2,3 g du composé attendu.
Rendement = 92%

### Exemple 4 : (7S)-3,4-diméthoxy-N-méthylbicyclo[4.2.0]octa-1,3,5-trién-7-amine D'après EP 0 534 859

On fait réagir l'amine obtenu à l'exemple 3 avec une quantité équimolaire de l'acide (d) camphosulfonique dans l'éthanol. Après évaporation sous vide du solvant, le sel est recristallisé une première fois dans l'acétate d'éthyle puis dans l'acétonitrile jusqu'à l'obtention de l'énantiomère cible avec une pureté optique supérieure à 99% (évaluation par HPLC sur colonne Chiralcel ^{®} OD).

### Exemple 5 : 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2H-3-benzazépin-2-one D'après EP 0 534 859

La solution de sel de (d) camphosulfonate obtenu à l'exemple 4 dans l'acétate d'éthyle est amenée à pH basique à l'aide d'hydroxyde de sodium puis la phase organique est séparée, lavée, séchée sue Na₂SO₄ et évaporée.

On porte ensuite au reflux pendant 18 heures un mélange composé de 5,6 g de carbonate de potassium, 2,2 g de l'amine ci-dessus dans 100 mL d'acétone et de 4g de 3-(3-iodopropyl)-7,8-diméthoxy-1,3-dihydro-2*H*-3-benzazépin-2-one.

On évapore le solvant sous vide, on reprend le résidu à l'acétate d'éthyle, puis on extrait à l'acide chlorhydrique 3N.

On amène la phase aqueuse décantée à pH basique à l'aide d'hydroxyde de sodium, puis on l'extrait à l'acétate d'éthyle. Après lavage à neutralité et séchage sur MgSO₄, on évapore sous vide pour obtenir 4,5 g d'une huile qui est purifiée sur colonne de silice en utilisant comme éluant un mélange dichlorométhane/méthanol (90/10).
Rendement = 64%

### Exemple 6 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one D'après EP 0 534 859

5 g du composé obtenu à l'exemple 5 dans 50 mL d'acide acétique glacial sont hydrogénés dans un appareil de Parr, sous un pression de 4,9 bar d'hydrogène à température ambiante pendant 24 heures, en présence d'1 g d'hydroxyde de palladium à 10%. On filtre le catalyseur, on évapore le solvant, puis on reprend le résidu sec à l'eau et à l'acétate d'éthyle. On sèche la phase organique sur sulfate de magnésium anhydre, on concentre sous vide puis le résidu est purifié sur colonne de silice en utilisant comme éluant un mélange dichlorométhane/méthanol (95/5). Après recristallisation dans l'acétate d'éthyle, on obtient 2g du composé attendu.
Rendement = 40%
P.F. = 101-103°C

## Revendications

1. Procédé de synthèse du composé de formule (I) : 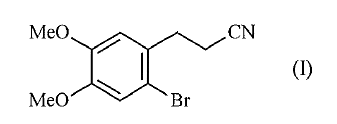 **caractérisé en ce que** le composé de formule (VIII) : 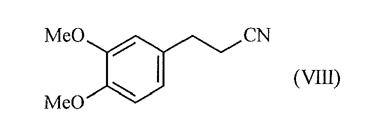 est soumis à l'action du *N*-bromosuccinimide en présence d'un solvant organique pour conduire au composé de formule (I).

2. Procédé selon la revendication 1, **caractérisé en ce que** le solvant organique utilisé pour effectuer la transformation du composé de formule (VIII) en composé de formule (I) est choisi parmi le *N*,*N*-diméthylformamide, le tétrahydrofurane, l'acétonitrile, l'acide acétique, le méthanol, le dichlorométhane et le toluène.

3. Procédé selon la revendication 2, **caractérisé en ce que** le solvant organique utilisé pour effectuer la transformation du composé de formule (VIII) en composé de formule (I) est le *N*,*N*-diméthylformamide.

4. Procédé selon l'une quelconque des revendications 1 à 3, **caractérisé en ce que** la transformation du composé de formule (VIII) en composé de formule (I) est conduite à une température comprise entre -10°C et 30°C.

5. Procédé selon la revendication 1, **caractérisé en ce que** le composé de formule (VIII) est préparé à partir du composé de formule (IX) : 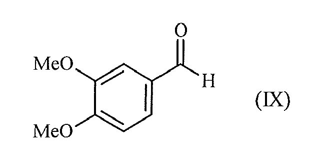 qui est transformé en composé de formule (X) : 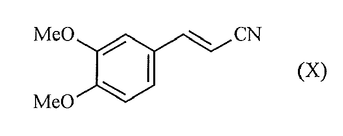 en présence d'un ylure de phosphore et d'une base dans un solvant organique,
lequel est transformé en composé de formule (VIII) : 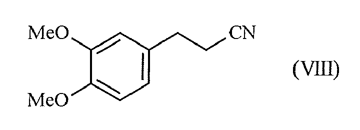 par une réaction de réduction en présence d'un agent donneur d'hydrure dans un solvant organique ou un mélange de solvants organiques.

6. Procédé selon la revendication 5, **caractérisé en ce que** l'ylure de phosphore utilisé pour effectuer la transformation du composé de formule (IX) en composé de formule (X) est le diéthylcyanométhylphosphonate ou le (triphénylphosphoranylidène)acétonitrile.

7. Procédé selon la revendication 6, **caractérisé en ce que** l'ylure de phosphore utilisé pour effectuer la transformation du composé de formule (IX) en composé de formule (X) est le diéthylcyanométhylphosphonate.

8. Procédé selon l'une quelconque des revendications 5 à 7, **caractérisé en ce que** la base utilisée pour effectuer la transformation du composé de formule (IX) en composé de
formule (X) est choisie parmi le *tert*-butylate de potassium, l'hydrure de sodium, la triéthylamine et l'hydrogénocarbonate de potassium.

9. Procédé selon la revendication 8, **caractérisé en ce que** la base utilisée pour effectuer la transformation du composé de formule (IX) en composé de formule (X) est le *tert-*butylate de potassium.

10. Procédé selon l'une quelconque des revendications 5 à 9, **caractérisé en ce que** le solvant organique utilisé pour effectuer la transformation du composé de formule (IX) en composé de formule (X) est choisi parmi le tétrahydrofurane, l'acétonitrile et le toluène.

11. Procédé selon la revendication 10, **caractérisé en ce que** le solvant organique utilisé pour effectuer la transformation du composé de formule (IX) en composé de formule (X) est le tétrahydrofurane.

12. Procédé selon l'une quelconque des revendications 5 à 11, **caractérisé en ce que** la transformation du composé de formule (IX) en composé de formule (X) est conduite à une température comprise entre -5°C et 120°C.

13. Procédé selon l'une quelconque des revendications 5 à 12, **caractérisé en ce que** l'agent donneur d'hydrure utilisé pour effectuer la transformation du composé de formule (X) en composé de formule (VIII) est choisi parmi le tétraborohydrure de sodium, le formate d'ammonium en présence de Pd/C et l'acide formique en présence de Pd(OAc)₂.

14. Procédé de synthèse selon la revendication 13, **caractérisé en ce que** l'agent donneur d'hydrure utilisé pour effectuer la transformation du composé de formule (X) en composé de formule (VIII) est le tétraborohydrure de sodium.

15. Procédé selon l'une quelconque des revendications 5 à 14, **caractérisé en ce que** le solvant organique utilisé pour effectuer la transformation du composé de formule (X) en composé de formule (VIII) est choisi parmi le méthanol, l'éthanol, le tétrahydrofurane ou le mélange pyridine/méthanol.

16. Procédé selon la revendication 15, **caractérisé en ce que** le solvant organique utilisé pour effectuer la transformation du composé de formule (X) en composé de formule (VIII) est le mélange pyridine/méthanol.

17. Procédé selon l'une quelconque des revendications 5 à 16, **caractérisé en ce que** la transformation du composé de formule (X) en composé de formule (VIII) est conduite à une température comprise entre 25°C et 110°C.

18. Procédé de synthèse de l'ivabradine, de ses sels pharmaceutiquement acceptables et de ses hydrates, **caractérisé en ce que** le composé de formule (VIII) est transformé en intermédiaire de formule (I) suivant le procédé de la revendication 1, puis l'intermédiaire de formule (I) est transformé en ivabradine, qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

## Patentansprüche

1. Verfahren zur Synthese der Verbindung der Formel (I): 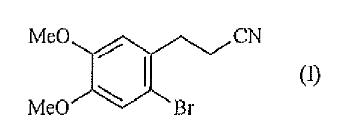 **dadurch gekennzeichnet, dass** die Verbindung der Formel (VIII): 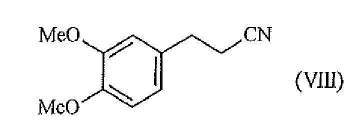 in Gegenwart eines organischen Lösungsmittels der Einwirkung von *N*-Bromsuccinimid ausgesetzt wird zur Bildung der Verbindung der Formel (I).

2. Verfahren nach Anspruch 1, **dadurch gekennzeichnet, dass** das zur Durchführung der Umwandlung der Verbindung der Formel (VIII) in die Verbindung der Formel (I) verwendete organische Lösungsmittel aus *N,N*-Dimethylformamid, Tetrahydrofuran, Acetonitril, Essigsäure, Methanol, Dichlormethan und Toluol ausgewählt wird.

3. Verfahren nach Anspruch 2, **dadurch gekennzeichnet, dass** das zur Durchführung der Umwandlung der Verbindung der Formel (VIII) in die Verbindung der Formel (I) verwendete organische Lösungsmittel *N,N*-Dimethylformamid ist.

4. Verfahren nach einem der Ansprüche 1 bis 3, **dadurch gekennzeichnet, dass** die Umwandlung der Verbindung der Formel (VIII) in die Verbindung der Formel (I) bei einer Temperatur zwischen -10 °C und 30 °C durchgeführt wird.

5. Verfahren nach Anspruch 1, **dadurch gekennzeichnet, dass** die Verbindung der Formel (VIII) hergestellt wird ausgehend von der Verbindung der Formel (IX): 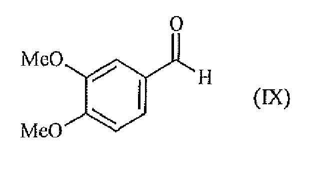 die in Gegenwart eines Phosphorylids und einer Base in einem organischen Lösungsmittel in die Verbindung der Formel (X) umgewandelt wird: 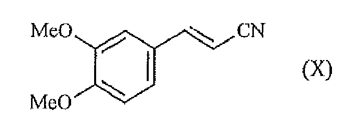 welche durch eine Reduktionsreaktion in Gegenwart eines Hydriddonors in einem organischen Lösungsmittel oder einer Mischung von organischen Lösungsmitteln in die Verbindung der Formel (VIII) umgewandelt wird:

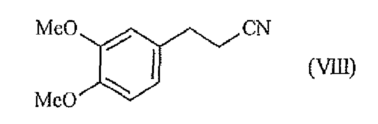

6. Verfahren nach Anspruch 5, **dadurch gekennzeichnet, dass** das zur Durchführung der Umwandlung der Verbindung der Formel (IX) in die Verbindung der Formel (X) verwendete Phosphorylid Diethylcyanomethylphosphonat oder (Triphenylphosphoranyliden)-acetonitril ist.

7. Verfahren nach Anspruch 6, **dadurch gekennzeichnet, dass** das zur Durchführung der Umwandlung der Verbindung der Formel (IX) in die Verbindung der Formel (X) verwendete Phosphorylid Diethylcyanomethylphosphonat ist.

8. Verfahren nach einem der Ansprüche 5 bis 7, **dadurch gekennzeichnet, dass** die zur Durchführung der Umwandlung der Verbindung der Formel (IX) in die Verbindung der Formel (X) verwendete Base aus Kalium-*tert*.-butylat, Natriumhydrid, Triethylamin und Kaliumhydrogencarbonat ausgewählt wird.

9. Verfahren nach Anspruch 8, **dadurch gekennzeichnet, dass** die zur Durchführung der Umwandlung der Verbindung der Formel (IX) in die Verbindung der Formel (X) verwendete Base Kalium-*tert*.-butylat ist.

10. Verfahren nach einem der Ansprüche 5 bis 9, **dadurch gekennzeichnet, dass** das zur Durchführung der Umwandlung der Verbindung der Formel (IX) in die Verbindung der Formel (X) verwendete organische Lösungsmittel aus Tetrahydrofuran, Acetonitril und Toluol ausgewählt wird.

11. Verfahren nach Anspruch 10, **dadurch gekennzeichnet, dass** das zur Durchführung der Umwandlung der Verbindung der Formel (IX) in die Verbindung der Formel (X) verwendete organische Lösungsmittel Tetrahydrofuran ist.

12. Verfahren nach einem der Ansprüche 5 bis 11, **dadurch gekennzeichnet, dass** die Umwandlung der Verbindung der Formel (IX) in die Verbindung der Formel (X) bei einer Temperatur zwischen -5 °C und 120 °C durchgeführt wird.

13. Verfahren nach einem der Ansprüche 5 bis 12, **dadurch gekennzeichnet, dass** der zur Durchführung der Umwandlung der Verbindung der Formel (X) in die Verbindung der Formel (VIII) verwendete Hydriddonor aus Natriumtetraborhydrid, Ammoniumformiat in Gegenwart von Pd/C und Ameisensäure in Gegenwart von Pd(OAc)₂ ausgewählt wird.

14. Verfahren zur Synthese nach Anspruch 13, **dadurch gekennzeichnet, dass** der zur Durchführung der Umwandlung der Verbindung der Formel (X) in die Verbindung der Formel (VIII) verwendete Hydriddonor Natriumtetraborhydrid ist.

15. Verfahren nach einem der Ansprüche 5 bis 14, **dadurch gekennzeichnet, dass** das zur Durchführung der Umwandlung der Verbindung der Formel (X) in die Verbindung der Formel (VIII) verwendete organische Lösungsmittel aus Methanol, Ethanol, Tetrahydrofuran oder einer Pyridin/Methanol-Mischung ausgewählt wird.

16. Verfahren nach Anspruch 15, **dadurch gekennzeichnet, dass** das zur Durchführung der Umwandlung der Verbindung der Formel (X) in die Verbindung der Formel (VIII) verwendete organische Lösungsmittel die Pyridin/Methanol-Mischung ist.

17. Verfahren nach einem der Ansprüche 5 bis 16, **dadurch gekennzeichnet, dass** die Umwandlung der Verbindung der Formel (X) in die Verbindung der Formel (VIII) bei einer Temperatur zwischen 25 °C und 110 °C durchgeführt wird.

18. Verfahren zur Synthese von Ivabradin, seinen pharmazeutisch annehmbaren Salzen und seinen Hydraten, **dadurch gekennzeichnet, dass** die Verbindung der Formel (VIII) nach der Verfahrensweise von Anspruch 1 in das Zwischenprodukt der Formel (I) umgewandelt wird, wonach das Zwischenprodukt der Formel (I) in Ivabradin umgewandelt wird, welches in seine Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und in ihre Hydrate umgewandelt werden kann.

## Claims

1. Process for the synthesis of the compound of formula (I): 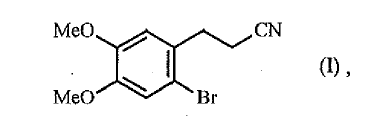 **characterised in that** the compound of formula (VIII): 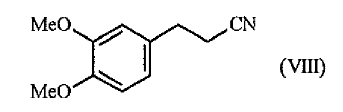 is subjected to the action of N-bromosuccinimide in the presence of an organic solvent to yield the compound of formula (I).

2. Process according to claim 1, **characterised in that** the organic solvent used to carry out the conversion of the compound of formula (VIII) into the compound of formula (I) is selected from *N,N*-dimethylformamide, tetrahydrofuran, acetonitrile, acetic acid, methanol, dichloromethane and toluene.

3. Process according to claim 2, **characterised in that** the organic solvent used to carry out the conversion of the compound of formula (VIII) into the compound of formula (I) is *N,N*-dimethylformamide.

4. Process according to any one of claims 1 to 3, **characterised in that** the conversion of the compound of formula (VIII) into the compound of formula (I) is carried out at a temperature between -10°C and 30°C, inclusive.

5. Process according to claim 1, **characterised in that** the compound of formula (VIII) is prepared starting from the compound of formula (IX): 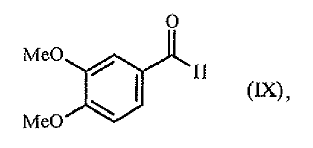 which is converted into the compound of formula (X): 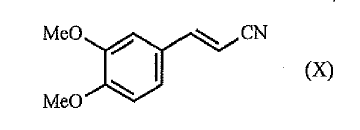 in the presence of a phosphorus ylide and a base in an organic solvent,
which is converted into the compound of formula (VIII): 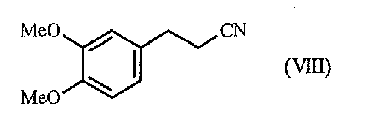 by a reduction reaction in the presence of a hydride donor agent in an organic solvent or mixture of organic solvents.

6. Process according to claim 5, **characterised in that** the phosphorus ylide used to carry out the conversion of the compound of formula (IX) into the compound of formula (X) is diethyl cyanomethyl phosphonate or (triphenylphosphoranylidene)acetonitrile.

7. Process according to claim 6, **characterised in that** the phosphorus ylide used to carry out the conversion of the compound of formula (IX) into the compound of formula (X) is diethyl cyanomethyl phosphonate.

8. Process according to any one of claims 5 to 7, **characterised in that** the base used to carry out the conversion of the compound of formula (IX) into the compound of formula (X) is selected from potassium *tert*-butoxide, sodium hydride, triethylamine and potassium hydrogen carbonate.

9. Process according to claim 8, **characterised in that** the base used to carry out the conversion of the compound of formula (IX) into the compound of formula (X) is potassium *tert*-butoxide.

10. Process according to any one of claims 5 to 9, **characterised in that** the organic solvent used to carry out the conversion of the compound of formula (IX) into the compound of formula (X) is selected from tetrahydrofuran, acetonitrile and toluene.

11. Process according to claim 10, **characterised in that** the organic solvent used to carry out the conversion of the compound of formula (IX) into the compound of formula (X) is tetrahydrofuran.

12. Process according to any one of claims 5 to 11, **characterised in that** the conversion of the compound of formula (IX) into the compound of formula (X) is carried out at a temperature between -5°C and 120°C, inclusive.

13. Process according to any one of claims 5 to 12, **characterised in that** the hydride donor agent used to carry out the conversion of the compound of formula (X) into the compound of formula (VIII) is selected from sodium borohydride, ammonium formate in the presence of Pd/C, and formic acid in the presence of Pd(OAc)₂.

14. Synthesis process according to claim 13, **characterised in that** the hydride donor agent used to carry out the conversion of the compound of formula (X) into the compound of formula (VIII) is sodium borohydride.

15. Process according to any one of claims 5 to 14, **characterised in that** the organic solvent used to carry out the conversion of the compound of formula (X) into the compound of formula (VIII) is selected from methanol, ethanol, tetrahydrofuran and the mixture pyridine/methanol.

16. Process according to claim 15, **characterised in that** the organic solvent used to carry out the conversion of the compound of formula (X) into the compound of formula (VIII) is the mixture pyridine/methanol.

17. Process according to any one of claims 5 to 16, **characterised in that** the conversion of the compound of formula (X) into the compound of formula (VIII) is carried out at a temperature between 25°C and 110°C, inclusive.

18. Process for the synthesis of ivabradine, pharmaceutically acceptable salts thereof and hydrates thereof, **characterised in that** the compound of formula (VIII) is converted into the intermediate of formula (I) according to the process of claim 1, and then the intermediate of formula (I) is converted into ivabradine, which may be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.
